# EUROPÄISCHE PATENTSCHRIFT

(11) **EP 3 852 610 B1**
(45) Veröffentlichungstag und Bekanntmachung des Hinweises auf die Patenterteilung: **14.09.2022**
(21) Anmeldenummer: 19773338.9
(22) Anmeldetag: 06.08.2019
(51) Int. Cl.: A61B 5/00, A61B 17/02, A61D 1/00, A61B 17/00

(54) **HAUTKAMMERSYSTEM FÜR DIE INTRAVITALMIKROSKOPIE**
SKINFOLD CHAMBER SYSTEM FOR INTRAVITAL MICROSCOPY
SYSTÈME DE CHAMBRE CUTANÉE POUR MICROSCOPIE INTRAVITALE

(30) Priorität: 18.09.2018 DE 102018007340
(43) Veröffentlichungstag der Anmeldung: 28.07.2021
(73) Patentinhaber: Johannes Gutenberg-Universität Mainz, 55122 Mainz (DE); Universitätsmedizin der Johannes Gutenberg-Universität Mainz, 55131 Mainz (DE)
(72) Erfinder: SCHRÖDER, Thies, 55268 Nieder-Olm (DE); HEIMANN, Axel, 55129 Mainz (DE); KÄFER, Katharina, 55252 Mainz-Kastel (DE); KRÜGER, Katja, 55252 Mainz-Kastel (DE); KEMPSKI, Oliver, 65343 Eltville am Rhein (DE)
(74) Vertreter: Rudolph, Ulrike
(86) Internationale Anmeldenummer: PCT/DE2019/000211
(87) Internationale Veröffentlichungsnummer: WO 2020/057679

(56) Entgegenhaltungen:
- WO-A1-2015/135992
- FENG-CHIEH LI ET AL: "Dorsal Skin Fold Chamber for High Resolution Multiphoton Imaging", OPTICAL AND QUANTUM ELECTRONICS, KLUWER ACADEMIC PUBLISHERS, BO, Bd. 37, Nr. 13-15, 1. Dezember 2005 (2005-12-01), Seiten 1439-1445, XP019261668, ISSN: 1572-817X
- LEHR HANS-ANTON ET AL: "Dorsal skinfold chamber technique for intravital microscopy in nude mice", AMERICAN JOURNAL OF PATHOLOGY; [10640], ELSEVIER INC, US, Bd. 143, Nr. 4, 1. Januar 1993 (1993-01-01), Seiten 1055-1062, XP002488189, ISSN: 0002-9440
- ZHANG ZHIWU ET AL: "Image-guided plasma therapy of cutaneous wound", PROGRESS IN BIOMEDICAL OPTICS AND IMAGING, SPIE - INTERNATIONAL SOCIETY FOR OPTICAL ENGINEERING, BELLINGHAM, WA, US, Bd. 8935, 26. Februar 2014 (2014-02-26), Seiten 89350I-89350I, XP060033584, ISSN: 1605-7422, DOI: 10.1117/12.2041092 ISBN: 978-1-5106-0027-0
- PAPENFUSS H D ET AL: "A transparent access chamber for the rat dorsal skin fold", MICROVASCULAR RESEARCH, ACADEMIC PRESS, US, Bd. 18, Nr. 3, 1. November 1979 (1979-11-01), Seiten 311-318, XP023033394, ISSN: 0026-2862, DOI: 10.1016/0026-2862(79)90039-6 [gefunden am 1979-11-01]

## Beschreibung

Die Erfindung betrifft ein Hautkammersystem für die Intravitalmikroskopie (IVM), d.h. die Mikroskopie im bzw. am lebenden Organismus.

Die Intravitalmikroskopie (IVM) wird in der biomedizinischen Forschung eingesetzt, um biologische dynamische Prozesse mit zellulärer und subzellulärer Auflösung in ihrer natürlichen Umgebung über eine Zeitspanne zu untersuchen. Mit der Intravitalmikroskopie (IVM) unter Verwendung eines Hautkammersystems, beispielsweise eines Rückenhautkammersystems (Dorsalkammersystems) kann z.B. die Migration und Proliferation von Zellen in der Unterhaut von insbesondere Mäusen oder Ratten über Tage bis Wochen innerhalb eines Tieres wiederholt mikroskopisch beobachtet, untersucht und visualisiert werden, ohne dass immer wieder operative Eingriffe vorgenommen werden müssen (vgl. Prunier et al., 2017).

Die im Stand der Technik bekannten Hautkammersysteme sind oft als Rückenhautkammersysteme realisiert und umfassen (vgl. z.B. WO2015/135992 A1, Palmer et al. 2011, Prunier et al. 2017, US 2018/0154073 A1, Sobolik et al. 2015) eine Rahmenvorrichtung (Rahmenstruktur) als Tragwerk für ein Hautstück (einen Hautbereich, Hautabschnitt). Die bekannten (Trag-)Rahmenvorrichtungen bestehen üblicherweise aus zwei komplementären und meist annähernd deckungsgleichen Platten, die eine fensterartige Öffnung (Ausnehmung) aufweisen und mit Abstand und spiegelbildlich zueinander angeordnet sind. Der durch den Abstand gebildete Zwischenraum dient zur Aufnahme des Hautstücks, nämlich einer Hautfalte, insbesondere einer Rückenhautfalte des Tieres, auf dem das Hautkammersystem montiert ist. Die beiden Platten sind üblicherweise durch ein Schrauben-Mutter-System miteinander verbunden, wobei die Schrauben durch vorgestanzte Löcher in der zwischen den beiden Platten eingeschlossenen Hautfalte hindurch geführt werden und so die Rahmenvorrichtung bzw. das (Rücken-) Hautkammersystem am Tier fixieren.

Ein solches Hautkammersystem mit einer Rahmenvorrichtunq, die aus zwei separaten Einzelrahmen besteht, welche mittels Befestigungselementen über Durchgangslöcher so verbunden werden, dass die Haut dazwischen eingepresst ist, ist auch aus Feng-Chieh Li et al. ("Dorsal Skin Fold Chamber for High Resolution Multiphoton Imaging", December 2005 Optical and Quantum Electronics, 37(13):1439-1445, DOI:10.1007/s11082-005-4223-4), Lehr, Hans-Anton et al. ("Dorsal skinfold chamber technique for intravital microscopy in nude mice", November 1993, American Journal Of Pathology 143(4):1055-62, ISSN: 0002-9440), Zhiwu Zhang et al. ("Image-guided plasma therapy of cutaneous wound", Proc. SPIE 8935, Advanced Biomedical and Clinical Diagnostic Systems XII, 89350I (27 February 2014); doi.org/10.1117/12.2041092) und Papenfuss H. D. et al. ("A transparent access chamber for the rat dorsal Skin fold", Microvasc. Res. 1979, 18(3):311-318; doi:10.1016/0026-2862(79)90039-6) bekannt.

In die fensterartige Öffnung/Ausnehmung der Platten ist eine durchsichtige Abdeckung einpassbar, so dass ein Beobachtungsfenster auf die zwischen den Platten der Rahmenvorrichtung eingespannte (Rücken-)Haut des betreffenden Tieres besteht. An dieser Stelle ist bzw. wird die Haut dieses Tieres präpariert. Das Beobachtungsfenster besteht beispielsweise aus Glas und ist beispielsweise mit einem Sprengring in der Öffnung/Ausnehmung befestigt. Durch das Beobachtungsfenster kann die präparierte Haut über mehrere Tage bis Wochen mikroskopisch dokumentierbar beobachtet werden.

Dieses Modell hat sich als äußerst nützlich erwiesen, um die einzelnen Schritte der Tumorentstehung besser zu verstehen, z.B. die Anregung des Blutgefäßwachstums (Angiogenese) oder frühe Schritte in der Metastasierung.

Das Model wurde auch bereits erfolgreich eingesetzt, um die Wirkung von Krebstherapien zu evaluieren und zu visualisieren. Die größte Schwäche der bekannten Haut- und insbesondere Rückenhautkammersysteme ist jedoch ihr schneller Verschleiß, da die Fensterkammer nach einigen Tagen undicht wird und Infektionen entstehen (können). Andere Probleme sind die Abriebbelastungen an den Befestigungslöchern in der Tierhaut, die die Stahl/Titan-Kammer halten müssen, das Herauswachsen von Tumoren an den Fensterrändern, das relativ hohe Gewicht der Kammer, der mit der Stahlkammer verbundene Temperaturverlust, künstliche Bedingungen im beobachteten Gewebe in Form des direkten und ständigen Kontakts mit dem Glasfenster, die unnatürliche Wachstumsform implantierter Tumoren zwischen den Beobachtungsfenstern, und Veränderungen im Verhalten der Tiere, die mit der unhandlichen Vorrichtung an ihrem Rücken bzw. Körper verbunden sind.

Selbst wenn die (Trag-)Rahmenvorrichtung aus so leichtem Material wie Titan bestehen, haben diese bekannten Hautkammersysteme immer noch den großen Nachteil, dass das Gesamtgewicht des Systems (Rahmen, Schrauben und Titanmuttern) relativ groß ist und - im Fall der Rückenhautkammersysteme - die auf dem Tierrücken mit ihren schmalen Seitenkanten quasi stehenden Platten bei Bewegung des Tieres vor allem lateral hin und her schaukeln, was insbesondere dort, wo die Befestigungsschrauben die Haut durchbohren, zu Hautschäden führt und das Tier auch im übrigen bei seinen Bewegungen behindern und belasten.

In der Praxis ermöglichen die bekannten Hautkammersysteme eine wiederholte direkte intravitale mikroskopische Beobachtung bei ein und demselben Versuchstier nicht länger als über einen Zeitraum von ca. 2 Wochen (vgl. Palmer et al. 2011).

Der vorliegenden Erfindung liegt die Aufgabe zugrunde, ein Hautkammersystem bereitzustellen, bei dem diese Nachteile vermieden sind.

Eine Lösung dieser Aufgabe besteht in der Angabe eines Hautkammersystems mit einer Rahmenvorrichtung (Rahmenstruktur, Rahmenstruktur), die als U-förmiger Klapprahmen mit einem U-förmigen äußeren Rahmenteil und einem U-förmigen inneren Rahmenteil realisiert ist. Diese beiden Rahmenteile sind an den offenen Enden ihrer U-Schenkel durch je eine Gelenkverbindung derart miteinander gekoppelt, dass das innere Rahmenteil gegen das äußere Rahmenteil klappbar (schwenkbar) ist, - vorzugsweise um wenigstens etwa 180° - so dass im eingeklappten (eingeschwenkten) Zustand das innere Rahmenteil innerhalb der U-Öffnung des äußeren Rahmenteils positionierbar bzw. positioniert ist, und im ausgeklappten (ausgeschwenkten) Zustand das innere Rahmenteil und das äußeren Rahmenteil spiegelbildlich zueinander liegen und bei einem Öffnungswinkel von 180° im Idealfall eine vierseitige Öffnung mit zwei Seitenpaaren aus jeweils sich gegenüberliegenden und annähernd gleich langen Seiten zwischen sich einschließen respektive umschließen. Das äußere Rahmenteil und das innere Rahmenteil haben jeweils im Querschnitt ein L-förmiges Profil, so dass jedes Rahmenteil eine bezüglich der Klapprahmenebene horizontale U-förmig verlaufende Rahmenwand und eine bezüglich der Klapprahmenebene vertikale U-förmig verlaufende Rahmenwand aufweist. Die beiden Rahmenteile sind derart zueinander ausgerichtet, dass in geschlossener Klapprahmenstellung ihre vertikalen Rahmenwände annähernd parallel zu einander verlaufen und ihre horizontalen Rahmenwände im Wesentlichen in der gleichen Ebene und gegenläufig zueinander ausgerichtet sind.

Der Querschnitt der beiden Rahmenteile in geschlossener Klapprahmenstellung zeigt somit, dass die beiden bezüglich der Klapprahmenebene vertikal verlaufenden L-Profilschenkel von einerseits äußerem und andererseits innerem Rahmenteil annähernd parallel zu einander verlaufen, und dass die beiden bezüglich der Klapprahmenebene horizontalen L-Profilschenkel von einerseits äußerem und andererseits innerem Rahmenteil im wesentlichen in der gleichen Ebene und gegenläufig zueinander ausgerichtet sind.

Das L-Profil von äußerem und innerem Rahmenteil erlaubt es, beim Einsetzen des Klapprahmens in die Haut des betreffenden Tieres (oder Menschen) sowohl die Hautränder am U-förmigen Hautlappen als auch die Hautränder entlang der Hautöffnung derart an den beiden U-förmigen Rahmenteilen anzulegen und zu befestigen, dass bei geschlossenem Klapprahmen ein direkter Kontakt zwischen den Wundrändern von Hautlappen und Hautöffnung verhindert wird, und die Haut an diesen Rändern nicht wieder zusammenwachsen kann, während der Klapprahmen geschlossen ist.

Die Rahmenwände des äußeren Rahmenteils und/oder des inneren Rahmenteils sollten an den Schenkelenden abgerundete Kanten aufweisen, um bei dem im Einsatz befindlichen Hautkammersystem Gewebereizungen und/oder-Verletzungen insbesondere beim Öffnen und Schließen des Klapprahmens zu minimieren.

Die Gelenkverbindung sollte vorzugsweise derart an den Enden der U Schenkel des inneren und des äußeren Rahmenteils angeordnet sein, dass beim Öffnen des Klapprahmens durch Ausklappen (Ausschwenken) des inneren Rahmenteils aus dem äußeren Rahmenteil oder beim oder Schließen des Klapprahmens durch Einklappen (Einschwenken) des inneren Rahmenteils in das äußeren Rahmenteil die Schenkelenden des inneren Rahmenteils die Schenkelenden und Rahmenwände des äußeren Rahmenteils nicht überkragen, d.h. nicht über diese hinaus geführt werden. Dadurch werden Beeinträchtigungen oder mechanische Reizungen des umliegenden Gewebes beim Öffnen oder Schließen des Klapprahmens vermieden.

In einer bevorzugten Ausführungsform des erfindungsgemäßen Hautkammersystems ist die Gelenkverbindung des Klapprahmens als Scharnier realisiert, das zwei Ösen und einen Verbindungsstift aufweist und an den beiden vertikalen Rahmenwänden von äußerem und innerem Rahmenteil ausgebildet ist. Die Ösen sind vorzugsweise jeweils als eine Lochbohrung in der vertikalen Rahmenwand des inneren Rahmenteils und in der vertikalen Rahmenwand des äußeren Rahmenteils und jeweils nahe der freien U-Schenkelenden realisiert. Der Verbindungsstift ist derart ausgebildet und in den beiden Ösen angeordnet, dass er nicht aus dem von den beiden Ösen gebildeten Stiftkanal herausragt. Damit wird bei dem Hautkammersystem im Einsatz eine Gewebereizung weiter minimiert.

In einer besonders bevorzugten Ausführungsform des erfindungsgemäßen Hautkammersystems weisen äußeres und/oder inneres Rahmenteil jeweils in ihrer horizontalen Rahmenwand Durchgangsbohrungen auf, die einzeln oder gruppiert, beispielsweise paarweise, angeordnet sind. Bei einer Fixierung (Immobilisierung) des Klapprahmens an bzw. in der Haut des Versuchstieres mittels intrakutaner Nähte sind diese Durchgangsbohrungen bzw. Löcher für die Nahtführung vorgesehen.

Das im Querschnitt L-förmige Profil von äußerem Rahmenteil und innerem Rahmenteil kann gleichschenklig oder ungleichschenklig sein, das heißt die vertikale und die horizontale Rahmenwand eines Rahmenteils können die gleiche Höhe (bzw. Tiefe) haben oder sich in ihrer Höhe (bzw. Tiefe) unterscheiden.

Auch die korrespondierenden (einander entsprechenden) Rahmenwände des inneren und äußeren Rahmenteils können die gleiche Höhe (bzw. Tiefe) haben oder sich in ihrer Höhe (bzw. Tiefe) unterscheiden.

In einer bevorzugten Ausführungsform des erfindungsgemäßen Hautkammersystems ist die vertikale Rahmenwand des inneren Rahmenteils des Klapprahmens höher als die vertikale Rahmenwand des äußeren Rahmenteils. Das hat den Vorteil, dass bei der Anwendung des Hautkammersystems am Tier (oder Menschen), wenn sich der Klapprahmen z.B. in dessen Rückenhaut eingesetzt befindet (vgl. Ausführungsbeispiel 2) und an seinem inneren Rahmenteil der präparativ (chirurgisch) erzeugte Hautlappen befestigt ist, das Herausklappen diese inneren Rahmenteils mit anliegendem Hautlappen zwecks Öffnung des Klapprahmens erleichtert wird. Damit werden die Beeinträchtigungen des Tieres (oder Menschen) weiter minimiert.

Insbesondere bei dieser bevorzugten Ausführungsform ist weiter vorzugsweise vorgesehen, dass in der horizontalen Rahmenwand des äußeren Rahmenteils und/oder des inneren Rahmenteils, jeweils in derjenigen Oberfläche, zur vertikalen Rahmenwand hinweist (d.h. die im Querschnitt zur L-Innenseite des L-Profils hinweist) etwa mittig und der U-Form folgend wenigstens eine längliche Vertiefung (Längsnut, Längsrille) ausgebildet ist. Das hat den Vorteil der Oberflächenvergrößerung und fördert das Anhaften des Unterhautgewebes nach dem Einsetzen des Hautkammersystems.

Falls Durchgangsbohrungen für eine Nahtführung vorgesehen sind, sind diese vorzugsweise in der/den länglichen Vertiefung(en) angeordnet.

In einer bevorzugten Ausführungsform des erfindungsgemäßen Hautkammersystems weist der Klapprahmen im Bereich seiner U-Basis an der horizontalen Rahmenwand des äußeren Rahmenteils (bei Betrachtung des Querschnitt dieses äußeren Rahmenteils somit in der Ebene des horizontalen L-Profilschenkels) eine Auskragung auf, die beispielsweise die Form einer Lasche haben kann. Diese Auskragung kann als Griff oder Hebel beim Schließen und Öffnen des Klapprahmens dienen, solange dieser noch nicht einem Tier (oder Menschen) eingesetzt ist. Bei der Anwendung des Hautkammersystems, wenn sich der Klapprahmen z.B. in der Rückenhaut eines Versuchstiers eingesetzt befindet (vgl. Ausführungsbeispiel 2), dient die Auskragung als stabilisierendes Wiederlager beim Öffnen des Klapprahmens durch Ausklappen des inneren Rahmenteils mit daran befestigtem Hautlappen.

Vorzugsweise ist an der vertikalen Rahmenwand des inneren Rahmenteils (bei Betrachtung des Querschnitt dieses inneren Rahmenteils somit in der Ebene des vertikalen L-Profilschenkels) ebenfalls eine Auskragung ausgebildet, die beispielsweise ebenfalls die Form einer Lasche haben kann. Diese Auskragung dient zum leichteren Greifen des inneren Rahmenteils insbesondere beim Herausklappen aus dem äußeren Rahmenteil, d.h. beim Öffnen des Klapprahmens. Um das Greifen des inneren Rahmenteils insbesondere mit einer Pinzette oder anderen Hilfsmitteln noch weiter zu erleichtern, wird vorgeschlagen, Verankerungsmittel für solche Hilfswerkzeuge in dieser Auskragung auszubilden, beispielsweise Sack- oder Durchgangsbohrungen.

Erfindungsgemäß können die Auskragungen des inneren und äußeren Rahmenteils im Bereich der U-Basis des Klapprahmens auch derart ausgebildet und ausgeformt sein, dass sie nach Art einer Griffkonstruktion zusammenwirken, so dass das innere Rahmenteil ohne besondere Hilfswerkzeuge, vorzugsweise unmittelbar von Hand, ausgeklappt und somit der Klapprahmen geöffnet werden kann.

Als Material für den Klapprahmen und insbesondere für das äußere und innere Rahmenteil und die Gelenkverbindung haben sich Edelstahl, Titan und biokompatible Kunststoffe, wie insbesondere Polyethylen (PE), Polyvinylchlorid (PVC), Acrylglas (PMMA), Polycarbonat (PC), jeweils einzeln oder Kombinationen davon in der Praxis gut bewährt.

In einer besonders bevorzugten Ausführungsform sind insbesondere inneres und äußeres Rahmenteil ganz oder überwiegend aus biokompatiblen Kunststoffen gefertigt. Dadurch wird das Gewicht der U-Rahmenteile reduziert und die Gewebeintegration noch weiter verbessert. Diese Gewichtsreduktion ist insbesondere bei der Anwendung in kleineren Säugetierspezies wie Mäusen ein wesentlicher Vorteil.

Grundsätzlich können in ein und demselben Klapprahmen mehrere verschiedene Materialien gleichzeitig zum Einsatz kommen, so dass Stabilität und Gewichtsminimierung gleichzeitig optimiert sind.

In einer weiteren bevorzugten Ausführungsform des erfindungsgemäßen Hautkammersystems mit Klapprahmen ist an denjenigen Oberflächen der horizontalen Rahmenwände und/oder der vertikalen Rahmenwände, die zur L-Innenseite des jeweiligen L-Profils im Querschnitt hinweisen, eine Beschichtung ausgebildet, die aus einem nicht-biologischen oder biologischen Material besteht, welches die Verhaftung zwischen Hautgewebe und Klapprahmenoberfläche fördert oder die immunologische Abstoßung des Rahmens verringert.

Diese Beschichtung kann derart realisiert sein, dass sie kurzfristig und ohne besonderen technischen Aufwand erneuerbar ist, oder sie kann als dauerhaft montierte Beschichtung an der Rahmenoberfläche ausgebildet sein.

Als Material für die kurzlebige Beschichtung wird bevorzugt Gewebekleber, insbesondere Fibrinkleber vorgeschlagen, der auf die Oberfläche der betreffenden Rahmenwand/ Rahmenwände aufgesprüht oder aufgestrichen werden kann. Eine solche Filmbeschichtung ist kurzfristig und einfach herzustellen und kann bzw. sollte von Zeit zu Zeit erneuert werden. Bei dieser Ausführungsvariante des erfindungsgemäßen Hautkammersystems wird die Fixierung der Kontaktflächen der Klapprahmenteile am Hautgewebe durch eine Kombination von chirurgischer Naht und Gewebekleber erreicht.

Für die dauerhaft montierte oder jedenfalls lang anhaftende Beschichtung (aber auch für die der regelmäßig erneuerungsbedürftigen Beschichtung) wird bevorzugt ein Material vorgeschlagen, das eine poröse Matrix auf der Basis von Polycarbonat und/oder Kalzium und/oder Kieselsäure und/oder von anderen geeigneten Materialien umfasst, wobei die Poren zu einem Netzwerk aus Hohlräumen miteinander verbunden sind ("Matrix mit offener Porosität"). Derartige Materialien sind bekanntermaßen dazu geeignet, das Einwachsen von Zellen in die Beschichtung zu fördern, das heißt zu stimulieren und zu unterstützen. Mit einer gezielt erzeugten Variation in der Größe der Poren und der Verbindungshohlräume (Kanäle) zwischen den Poren kann das Einwachsen eines differenzierten Gefäßsystems mit unterschiedlich dicken und daher unterschiedlich differenzierten Blutgefäßen gefördert werden.

Der Einsatz eines erfindungsgemäßen Hautkammersystems mit U-förmigem Klapprahmen, dessen inneres und/oder äußeres Rahmenteil eine erfindungsgemäße Beschichtung aufweist, bietet den Vorteil, dass die beschichteten Rahmenteile zwar initial mit Hilfe einer Subkutan-Naht in bzw. an der Haut des Tieres (oder Menschen) verankert werden, dass diese Subkutan-Nahtfäden aber nach ca. 1-2 Wochen wieder entfernt werden können, weil nach dieser Zeitspanne der Rahmen erfahrungsgemäß ausreichend fest mit dem umliegenden Hautgewebe verbunden bzw. verwachsen ist.

Das erfindungsgemäße Hautkammersystem mit U-förmigem Klapprahmen ermöglicht insbesondere bei einem Versuchstier die Stabilisierung eines Hautlappens für eine wiederholte, langfristige multimodale Darstellung der subkutanen Haut und für einen wiederholten und langfristigen optischen und physikalischen Zugang zu experimentellen Tumoren oder Objekten, die in die Haut implantiert werden. Der optische Zugang umfasst diverse Methoden der Mikroskopie, von der regulären, zweidimensionalen Hellfeld- und Fluoreszenzmikroskopie bis hin zu dreidimensionalen Techniken wie der konfokalen oder der Zwei-Photonen-Mikroskopie. Dieses Hautkammersystem ist hervorragend geeignet für die mikroskopische Beobachtung und Erforschung von subkutan implantierten Materialien, Zellen, Tumoren und Sensoren in-vivo und in-situ, und es ist prinzipiell bei allen Tierarten mit mehrschichtiger Haut und auch beim Menschen anwendbar.

Das erfindungsgemäße Hautkammersystem wird einem Versuchstier vorzugsweise unter Anwendung eines Verfahrens eingesetzt, das die folgenden Schritte umfasst, die alle unter sterilen Bedingungen durchgeführt werden sollten:
Auf der fellfreien (d.h. primär felllosen oder vom Fell befreiten) Haut des anästhesierten und analgenierten Versuchstiers wird eine U-förmige Inzisionslinie mit den Dimensionen des einzusetzenden Klapprahmens markiert. Entlang dieser U-förmigen Markierungslinie (Markerlinie) wird ein Schnitt, beispielsweise ein Skalpellschnitt durch die Haut geführt, dessen Tiefe von der Oberhaut (Epidermis) bis durch die Unterschaut (Subkutis) hindurch reicht, nicht aber durch die darunter liegenden Faszien- und Muskelschichten. Bei der Schnittführung entlang der U-förmigen Markierungslinie wird vorzugsweise derart verfahren, dass die Schnittlinie (Schnittspur) entlang der U-Schenkel von der U-Basis aus betrachtet zunächst in bzw. parallel zu den U-Schenkel-Markierungslinien verläuft und im Endabschnitt jedes U-Schenkels leicht abgewinkelt von diesem und der U-Öffnung weggeführt wird, so dass der Abstand zwischen den beiden einander gegenüberliegenden Enden der Schnittlinie weiter ist als der Abstand zwischen den U-Schenkelenden der Markierungslinie. Der Abstand zwischen den beiden Enden der Schnittlinie sollte etwa so weit sein wie der Abstand zwischen den freien Kanten der horizontalen Rahmenwände des äußeren Rahmenteils, damit nach dem Einsetzten des Klapprahmens die Enden der U-Schenkel des inneren und äußeren Rahmenteils mit den dort angeordneten Gelenkverbindungen von Haut bedeckt sind und nicht von der Schnittnaht.

Dadurch wird deren Lage am bzw. im Tier stabilisiert und einem "Heraushebeln" entgegengewirkt.

Der auf diese Weise vorpräparierte U-förmige Hautlappen wird in der Ebene der Unterhautschicht, an der Grenze zwischen Unterhaut und darunter liegenden Faszienschichten, chirurgisch von dem darunterliegenden Gewebe abgetrennt (freipräpariert) und zurück geklappt, d.h. aus der umliegenden Haut des Tierrückens nach distal herausgeklappt.

In die entstandene Hautöffnung am Tierrücken wird der zuvor sterilisierte U-förmige Klapprahmen derart eingesetzt, dass das äußere Rahmenteil in der Hautöffnung platziert ist und das innere Rahmenteil in Offen-Stellung am zurück geklappten Hautlappen anlegbar ist. Mittels intrakutaner Nähte werden das innere Rahmenteil am Hautlappen und das äußere Rahmenteil am angrenzenden Gewebe der Haut um die Hautöffnung am/im Tierrücken befestigt. Zur Fixierung des Hautlappens am inneren Rahmenteil werden die Nahtfäden vorzugsweise von proximal (d.h. von "unter der Subkutis") U-förmig durch die Kutis geführt, durch die jeweils nebeneinander liegenden Löcher in der horizontalen Rahmenwand des inneren Rahmenteils gezogen und auf der freien, dem äußeren Rahmenteil zugewandten Oberfläche der Rahmenwand zusammengezogen und verknotet. Die Durchstichstellen in dem Hautlappen sollten dabei so platziert sein, dass beim Festziehen der Naht die Haut leicht zur Rahmenwand hingezogen wird. Vorzugsweise erfolgt nach dem Festziehen der Nahten noch ein Verkleben der Auflagefläche der Haut an der jeweils angrenzenden Oberfläche der Rahmenwand mittels eines handelsüblichen Gewebeklebers, beispielsweise Cyanacrylat (z.B. Histoacryl ^{™}).

Zum Schließen des Klapprahmens wird das innere Rahmenteil mit daran befestigtem Hautlappen in das in der Wundöffnung im Tierrücken fixierte äußere Rahmenteil eingeklappt. Vorzugsweise werden die Wundränder noch mit Sprühpflaster versiegelt.

Um die Lage der U-Schenkelenden des Klapprahmens im Tierrücken zu stabilisieren, kann eine Schutznaht oberhalb der Gelenkverbindung ausgebildet werden. Vorzugsweise werden hierfür an den Schenkelenden des U-förmigen Schnittkanals die beiden korrespondierenden Schnittkanten an Hautöffnung und Hautlappen über den (d.h. distal der) U-Schenkelenden des Klapprahmens im Bereich der Gelenkverbindung mittels chirurgischem Faden mit einer einfachen Naht vernäht.

Um ein Zusammenwachsen der Haut über den Klapprahmen hinweg zu verhindern, wird vorgeschlagen, vor dem Schließen des Klapprahmens, d.h. vor dem Einklappen des inneren Rahmenteils mit daran fixiertem Hautlappen in das äußere Rahmenteil, auf der vertikalen Rahmenwand des inneren Rahmenteils, insbesondere an dessen zur U-Öffnung des Klapprahmens hinweisenden Oberfläche oder an dessen zur vertikalen Rahmenwand des äußeren Rahmenteils hinweisenden Oberfläche oder an beiden ein (zuvor sterilisiertes) flexibles Band (z.B. ein Kunststoffband aus Polytetrafluorethylen/PTFE wie das im Handel erhältliche Teflon^{™}) anzuordnen. Nach dem Schließen des Klapprahmens sollte der nach außen überstehende Teil dieses Bandes abgeschnitten werden, um zu verhindern, dass das Versuchstier dieses Band wieder herausreißt und dadurch den Deckel öffnet.

Das eingesetzte Hautkammersystem sollte in regelmäßigen Abständen (vorzugsweise mindestens zweimal pro Woche) geöffnet werden, um ein Wiederzusammenwachsen des frei präparierten Hautlappens mit dem Gewebe in der Hautöffnung im/am Tierrücken zu verhindern. Dabei können mikroskopische und sonstige Beobachtungen sowie Manipulationen vorgenommen werden.

Für mikroskopische Beobachtungen und Untersuchungen des subkutanen Gewebes an der Unterseite des Hautlappens wird das Tier betäubt und der Klapprahmen wird langsam geöffnet, wobei nachgewachsenes Gewebe durch vorsichtige, stumpfe Abtrennung (beispielsweise mit einer stumpfen Schere oder Klemme) entfernt wird. Anschließend wird das Tier auf einem Heiztisch unter einem Mikroskop platziert. Das innere Rahmenteil mit dem Hautlappen wird derart auf dem Heiztisch angeordnet, dass die subkutane Gewebeschicht zum Beobachter bzw. zum Beobachtungsobjektiv hinweist. Um das Sichtfeld für die Mikroskopie zu ebnen, kann ein Deckglas auf die subkutane Haut gelegt werden.

In die subkutane Gewebeschicht des Hautlappens können nun Testobjekte, insbesondere Materialien oder Zellen oder Tumorgewebestücke implantiert und einer Langzeitbeobachtung unter annähernd natürlichen physiologischen und morphologischen Bedingungen unterworfen werden.

Das erfindungsgemäße Hautkammersystem erlaubt im Fall von implantiertem Tumorgewebe die mikroskopische Beobachtung von Umbauvorgängen wie z.B. Gefäßwachstum und Blutfluss in experimentell erzeugten Tumoren über mehrere Wochen oder sogar Monate. Diese Beobachtungen ermöglichen die Gewinnung von wichtigen neuen Erkenntnissen für ein besseres Verständnis und eine bessere Beschreibung wesentlicher Schritte in der Tumorentwicklung. Hierzu gehören insbesondere auch Tumorinvasion und Metastasierung, die beide das klinische Bild eines Patienten bestimmen und ein auf die klinische Situation abgestimmtes Therapieschema definieren.

Mit dem erfindungsgemäßen Hautkammersystem ist es zudem erstmals möglich, die Reaktionen der Tumormikroumgebung auf die Therapie nach längerer Zeit des Tumorwachstums, d.h. nach mehreren Wochen, Monaten oder sogar Jahren, zu visualisieren. Diese neue Möglichkeit stellt einen besonders bedeutenden Vorteil der Erfindung dar, weil sich menschliche Tumore in der Regel über einen längeren Zeitraum als ein Jahr entwickeln. Für Untersuchungen am Tiermodell mit Implantationszeiten von erheblich über einem Jahr empfiehlt sich die Verwendung einer langlebigeren Versuchstierspezies als Maus oder Ratte, die jeweils nur etwa ein Jahr lang leben.

Das erfindungsgemäße Hautkammersystem bietet ebenfalls erstmals die Möglichkeit, die Wechselwirkungen von implantierten Materialien mit dem einbettenden Gewebe über einen Zeitraum von Monaten oder gar Jahren mikroskopisch zu beobachten. Da viele implantierte Materialien für mehrere Jahre im menschlichen Körper verbleiben, besteht der Bedarf, genaue Vorhersagen über die Leistungsfähigkeit von implantierten Materialien und Strukturen in lebenden Organismen treffen zu können. Diesem Bedarf wird mit der vorliegenden Erfindung entsprochen. Das neue erfindungsgemäße Hautkammersystem bietet einen gegenüber herkömmlichen Hautkammersystemen wesentlich erweiterten Anwendungsbereich, nämlich eine erheblich längere zeitliche Nutzungsdauer (im Einsatz in der Haut), eine Vergrößerung der messbaren Fläche und das Einbringen von Sensoren zur Erfassung verschiedener Parameter, auch mit dauerhaftem Zugang bei geschlossenem Kammersystem.

Das erfindungsgemäße Hautkammersystem ermöglicht erstmals die Beobachtung und Messung der Interaktion von oral, intravenös, oder anderweitig verabreichten Testsubstanzen, wie z.B. Toxinen, Nahrungsstoffen und neuen Medikamenten, mit dem Gewebe in vivo und in Echtzeit. Durch den direkten, barrierefreien Zugang zum Gewebe bei weitgehender Abwesenheit von Wunden und anderen chirurgischen Artefakten wird hier, im Vergleich mit anderen verfügbaren Hautkammersystemen oder Gewebefenstern, eine weitgehend natürliche Testsituation in einem Versuchstier oder Probanden geschaffen. Interaktionen von Testsubstanzen mit dem Gewebe können einerseits mikroskopisch, andererseits durch andere geeignete, optische und nicht optische Detektionsmethoden, wie z.B. Surface-Enhanced Rahman Spectrosopy (SERS), beobachtet und gemessen werden.

Die Erfindung betrifft deshalb auch die Anwendung des erfindungsgemäßen Hautkammersystems in einem mikroskopischen oder nicht-mikroskopischen Verfahren zur Beobachtung, Überwachung, Detektion und/oder Messung von physiologischen Zuständen in vivo, insbesondere nach erfolgter Implantation von Tumoren und/oder Geweben und/oder Sensoren und/oder anderen Materialien in die Haut eines Säugerorganismus.

Im Folgenden wird die Erfindung anhand von Ausführungsbeispielen mit Figuren näher erläutert. Bei den Figuren zeigen:
- **Fig.1:**: eine perspektivische Ansicht auf einen erfindungsgemäßen Klapprahmen in **(A)** nahezu vollständig geöffnetem und **(B)** annähernd geschlossenem Zustand;
- **Fig. 2 :**: eine Aufsicht auf den Klapprahmen gemäß Fig.l, **(A)** im geschlossenen Zustand und **(B), (C)** im um 180° geöffneten Zustand; **(C)** zeigt die Rückseite des Klapprahmens gemäß **(B);**
- **Fig. 3 :**: eine Aufsicht auf die U-Basis des Klapprahmens gemäß Fig. 2 in **(A)** geschlossenem Zustand und **(B)** leicht geöffnetem Zustand;;
- **Fig. 4 :**: eine Seitenansicht des Klapprahmens gemäß Fig. 2 in **(A)** geschlossenem Zustand und **(B)** leicht geöffnetem Zustand;
- **Fig. 5 :**: den Querschnitt durch die U-Schenkel von innerem und äußerem U-Rahmenteil des Klapprahmens entlang der Schnittlinien V-V in Fig. 2, in vergrößertem Maßstab;
- **Fig. 6 :**: den Querschnitt durch inneres und äußeres U-Rahmenteil im Bereich der U-Basis entlang der Schnittlinien VI-VI in Fig. 2, in vergrößertem Maßstab;
- **Fig. 7 :**: zwei schematische Darstellungen eines Versuchstiers mit eingesetztem Klapprahmen und am inneren U-Rahmenteil fixiertem Hautlappen in geöffnetem Zustand, wobei in der Unterhaut des Hautlappens Testobjekte implantiert sind, nämlich in **(A)** verschiedene Testmaterialien und in **(B)** Tumorgewebe.

In den Figuren haben die nummerischen Bezugszeichen die folgenden Bedeutungen:
- 1 =: erfindungsgemäßer U-förmiger Klapprahmen des erfindungsgemäßen Hautkammersystems
- 2 =: U-Basis
- 3 =: äußeres U-Rahmenteil
- 4 =: laschenartige Auskragung des äußeren U-Rahmenteils
- 5 =: inneres U-Rahmenteil
- 6 =: laschenartige Auskragung des inneren U-Rahmenteils
- 7 =: Gelenkverbindung
- 8 =: Klapprahmenebene
- 16 =: Löcher (für die Nahtführung)
- 18 =: Längsnut (Längsrille, längliche Vertiefung)
- 20 =: L-Innenseite
- 21 =: Verbindungsstift
- 22 =: horizontale Rahmenwand des äußeren U-Rahmenteils
- 24 =: horizontale Rahmenwand des inneren U-Rahmenteils
- 25 =: vertikale Rahmenwand des äußeren U-Rahmenteils
- 27 =: vertikale Rahmenwand des inneren U-Rahmenteils
- 30 =: Oberfläche der horizontalen Rahmenwand des äußeren U-Rahmenteils
- 32 =: Oberfläche der horizontalen Rahmenwand des inneren U-Rahmenteils
- 35 =: Oberfläche der vertikalen Rahmenwand des äußeren U-Rahmenteils
- 37 =: Oberfläche der vertikalen Rahmenwand des inneren U-Rahmenteils

### Beispiel 1: Konstruktion eines erfindungsgemäßen Klapprahmens des erfindungsgemäßen Hautkammersystems

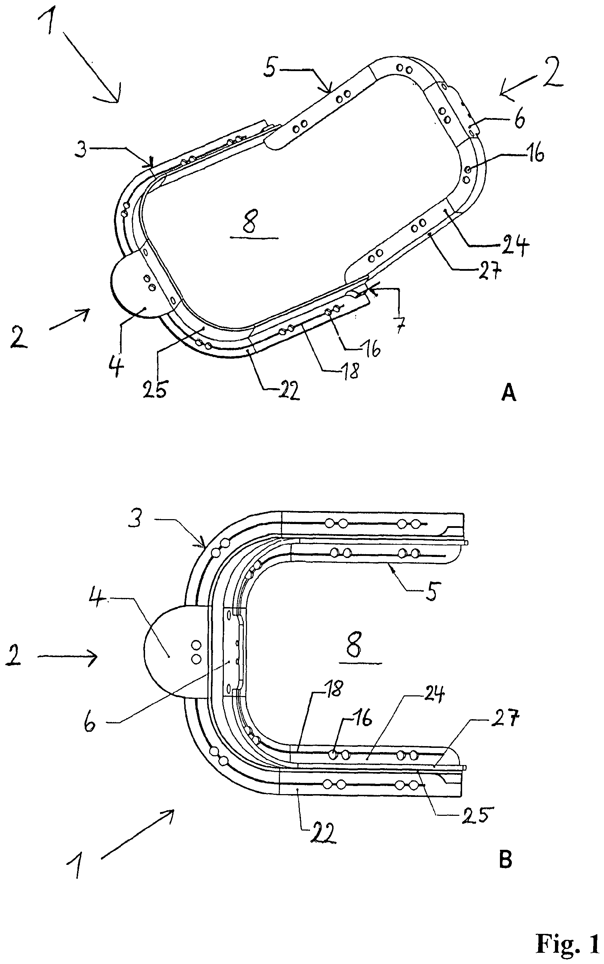

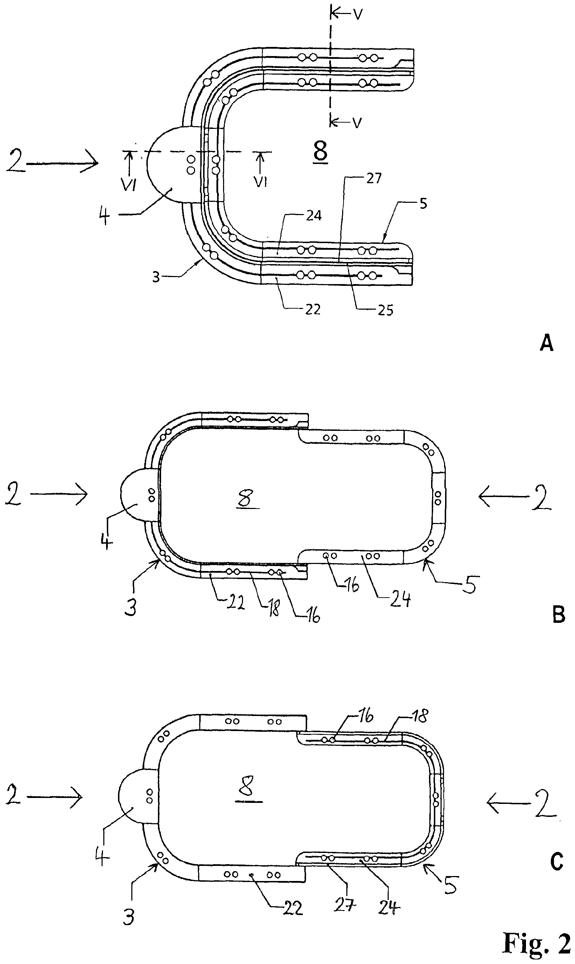

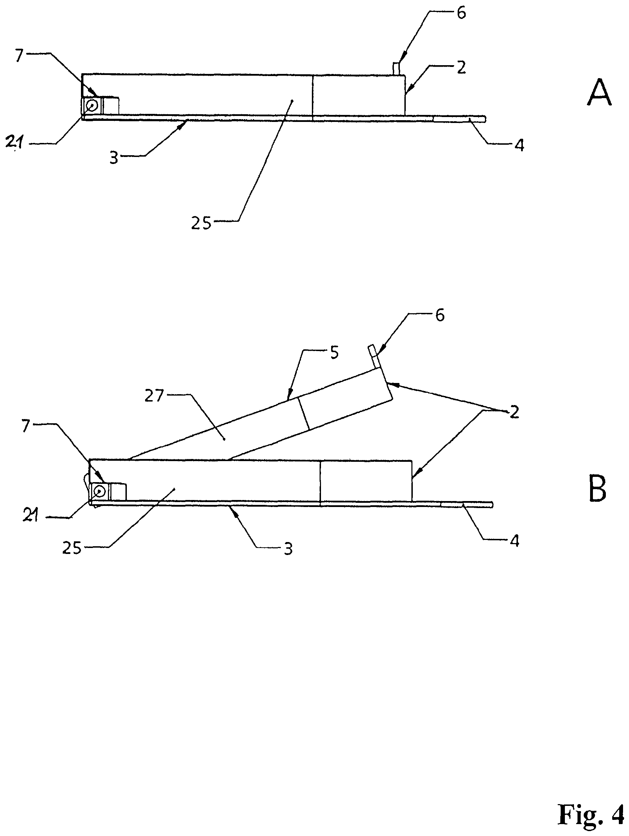

Eine erfindungsgemäße Klapprahmenkonstruktion ist in den Figuren 1 bis 4 dargestellt. Der U-förmige Klapprahmen 1 umfasst ein U-förmiges äußeres Rahmenteil 3 und ein U-förmiges inneres Rahmenteil 5. Diese beiden U-Rahmenteile 3, 5 sind an den offenen Enden Ihrer U-Schenkel durch je eine Gelenkverbindung 7 derart miteinander gekoppelt, dass das innere U-Rahmenteil 5 um vorzugsweise etwa 180° gegen das äußere U-Rahmenteil 3 klappbar ist, so dass in der einen Endposition ("geschlossener Zustand)" das innere U-Rahmenteil 5 innerhalb der U-Öffnung des äußeren U-Rahmenteils 3 positioniert ist (vgl. Fig. 1 B und Fig. 2 A), und in der anderen Endposition ("vollständig geöffneter Zustand") das innere U-Rahmenteil 5 und das äußere U-Rahmenteil 3 einander annähernd diametral gegenüber liegen und eine gemeinsame, annähernd rechteckige Öffnung zwischen sich einschließen (vgl. Fig. 1 A und Fig. 2 B, C). Jede der beiden langen Seiten dieser Öffnung wird von einem U-Schenkel des äußeren U-Rahmenteils 3 und dem damit gelenkig verbundenen U-Schenkel des inneren U-Rahmenteils 5 gebildete, und die beiden kurzen Seiten der Öffnung werden von der U-Basis des äußeren U-Rahmenteils 3 und gegenüber liegend von der U-Basis des inneren Rahmenteils 5 gebildet.

Die Gelenkverbindung 7 ermöglicht somit ein Einklappen und Ausklappen des inneren U-Rahmenteils 5 in die bzw. aus der U-Öffnung des äußeren U-Rahmenteils 3 und damit ein Öffnen und Schließen des Klapprahmens 1.

Die Gelenkverbindung 7, beispielsweise eine Scharnier, ist derart an den Enden der U-Schenkel des inneren und des äußeren U-Rahmenteils 5, 3 angeordnet, dass beim Öffnen oder Schließen des Klapprahmens 1, d.h. beim Ausklappen des inneren U-Rahmenteils 5 aus dem äußeren U-Rahmenteil 3 oder beim Einklappen des inneren U-Rahmenteils 5 in das äußeren U-Rahmenteil 3, die Schenkelenden des inneren U-Rahmenteils 5 nicht über die Schenkelenden und Rahmenwände 22, 25 des äußeren U-Rahmenteils 3 hinaus geführt werden.

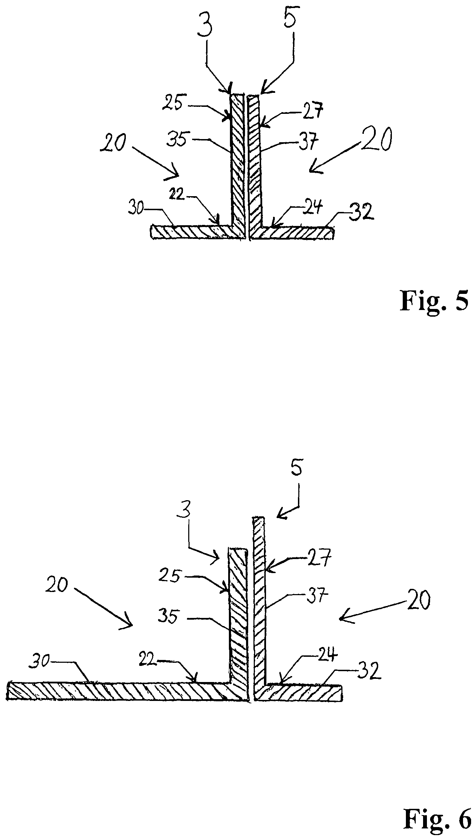

Äußeres U-Rahmenteil 3 und inneres U-Rahmenteil 5 haben jeweils im Querschnitt ein L-förmiges Profil (siehe Fig. 5), das derart ausgerichtet ist, dass die bezüglich der Klapprahmenebene 8 vertikalen L-Profilschenkel von äußerem und innerem U-Rahmenteil 3, 5 annähernd parallel, quasi Rücken an Rücken, zueinander verlaufen, und dass die bezüglich der Klapprahmenebene 8 horizontalen L-Profilschenkel von äußerem und innerem U-Rahmenteil 3, 5 in geschlossener Klapprahmenstellung im wesentlichen in der gleichen Ebene und gegenläufig, im Winkel von (genau oder annähernd) 180° zueinander ausgerichtet sind. Somit weist jedes U-Rahmenteil 3, 5 eine bezüglich der Klapprahmenebene 8 horizontale U-förmig verlaufende Rahmenwand 22, 24 und eine bezüglich der Klapprahmenebene 8 vertikale U-förmig verlaufende Rahmenwand 25, 27 auf (vgl. Fig. 1). An den Schenkelenden sind die Kanten dieser Rahmenwände 22, 24, 25, 27 vorzugsweise abgerundet.

Die Gelenkverbindung 7 zwischen äußerem und innerem U-Rahmenteil 3, 5 ist an den beiden vertikalen Rahmenwänden 25, 27 von äußerem und innerem U-Rahmenteil 3, 5 angeordnet. Sie kann, wie hier im Beispiel gemäß Fig. 1 bis Fig. 4, als Scharnier mit zwei Ösen und einem Verbindungsstift 21 realisiert sein. Hierfür sind die Ösen jeweils als eine Lochbohrung in der vertikalen Rahmenwand 27 des inneren U-Rahmenteils 5 und in der vertikalen Rahmenwand 25 des äußeren U-Rahmenteils 3 jeweils nahe der freien U-Schenkelenden realisiert. Der Verbindungsstift 21 ist so ausgebildet, dass er nicht über den Stiftkanal hinaus ragt, um Gewebereizungen zu minimieren.

Die Bemaßung des erfindungsgemäßen U-förmigen Klapprahmens 1 ist vorzugsweise die folgende:
Das äußere U-Rahmenteil 3 hat eine Gesamtlänge (von U-Basis bis zum freien U-Schenkelende) in der Größenordnung von 31 mm und eine Gesamtbreite (vom freien Rand der horizontalen Seitenwand des einen freien U-Schenkels bis zum freien Rand der horizontalen Seitenwand des anderen freien U-Schenkels) in der Größenordnung von 31 mm. Seine vertikale Rahmenwand 25 hat eine Höhe in der Größenordnung von 1 bis 4 mm und eine Dicke in der Größenordnung von 0,5 mm. Seine horizontale Rahmenwand 22 hat eine Höhe bzw. Tiefe in der Größenordnung von 5 mm und eine Dicke in der Größenordnung von 0,5 mm.

Das innere U-Rahmenteil 5 hat eine Gesamtlänge (von U-Basis bis zum freien U-Schenkelende) in der Größenordnung von 27,5 mm und eine Gesamtbreite (vom freien Rand der horizontalen Seitenwand des einen freien U-Schenkels bis zum freien Rand der horizontalen Seitenwand des anderen freien U-Schenkels) in der Größenordnung von 25 mm. Seine vertikale Rahmenwand 27 hat eine Höhe in der Größenordnung von 3 mm und eine Dicke in der Größenordnung von 0,5 mm. Seine horizontale Rahmenwand 24 hat eine Höhe bzw. Tiefe in der Größenordnung von 2 bis 5 mm und eine Dicke in der Größenordnung von 0,5 mm.

In einer besonders bevorzugten Ausführungsform (siehe Fig. 1, Fig. 2, Fig. 3) weisen äußeres und inneres U-Rahmenteil 3, 5 jeweils in ihrer horizontalen Rahmenwand 22, 24 kleine Löcher (Durchgangsbohrungen) 16 von beispielsweise ca. 1 mm Durchmesser auf, die einzeln oder z.B. paarweise gruppiert sind. Diese Löcher 16 dienen der Nahtführung bei der Fixierung (Immobilisierung) des Klapprahmens 1 an bzw. in der Haut des Versuchstieres (oder Menschen) mittels intrakutaner Nähte.

An der U-Basis 2 des Klapprahmens 1 weist das äußere U-Rahmenteil 3 an seiner horizontalen Rahmenwand 22 (d.h. in der Ebene des horizontalen L-Profilschenkels) eine laschenartige Auskragung 4 auf (vgl. Fig. 1 bis Fig. 4).

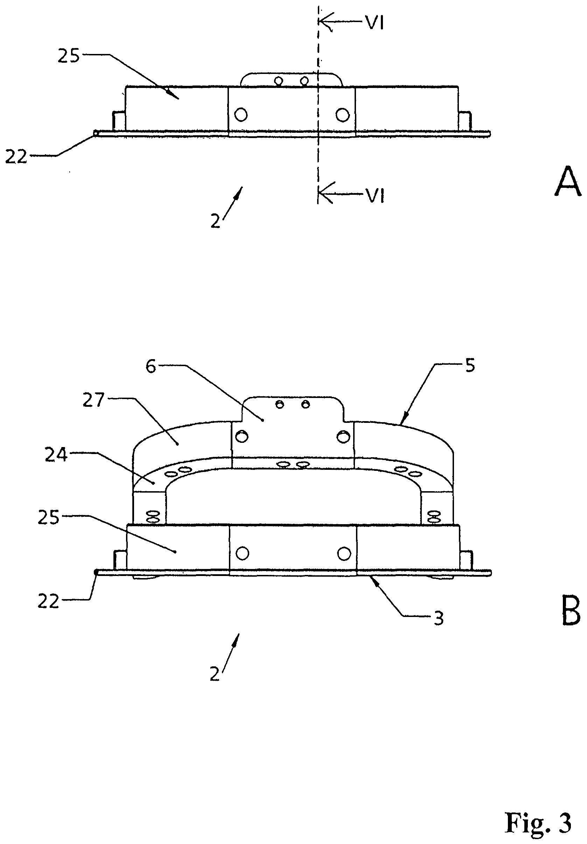

Ebenfalls an der U-Basis 2 des Klapprahmens 1 weist das innere U-Rahmenteil 5 an seiner vertikalen Rahmenwand 27 (d.h. in der Ebene des vertikalen L-Profilschenkels) eine laschenartige Auskragung 6 auf (vgl. Fig. 1 A und Fig. 3). In oder an dieser Auskragung 6 können Vertiefungen ausgebildet sein, beispielsweise kleinlumige Löcher (Durchgangsbohrungen oder Sackbohrungen), an denen ein Hilfswerkzeug zum Herausklappen dieses inneren U-Rahmenteils 5 zwecks Öffnens des Klapprahmens 1 angreifen bzw. angesetzt (befestigt) werden kann.

Alternativ können an der U-Basis 2 des Klapprahmens 1 jeweils am inneren und äußeren U-Rahmenteil 5, 3 Auskragungen derart ausgebildet sein, dass sie nach Art einer Griffkonstruktion zusammenwirken, so dass das innere U-Rahmenteil 5 ohne besondere Hilfswerkzeuge, vorzugsweise unmittelbar von Hand, ausgeklappt und somit der Klapprahmen 1 geöffnet werden kann.

Eine solche Griffkonstruktion kann z.B. derart realisiert sein, dass die beiden Auskragungen am inneren und äußeren U-Rahmenteil 5, 3 im Längsschnitt (parallel zur U-Symmetrieachse) jeweils ein L-Profil aufweisen und derart zueinander ausgerichtet sind, dass die beiden von der U-Basis ausgehenden L-Profilschenkel parallel quasi Rücken an Rücken zueinander verlaufen, und dass die jeweils davon abgewinkelten L-Profilschenkel (mit freiem Kopfende) in geschlossener Klapprahmenstellung im wesentlichen in der gleichen Ebene und entgegengesetzt im Winkel von (genau oder annähernd) 180° zueinander ausgerichtet sind, sodass sie im Profil zusammen ein "T" bilden.

Die vertikalen Rahmenwände 25, 27 des äußeren und des inneren U-Rahmenteils 3, 5 können sich in ihrer Höhe bzw. Tiefe unterscheiden.

In einer bevorzugten Ausführungsform ist die vertikale Rahmenwand 27 des inneren U-Rahmenteils 5 höher als die vertikale Rahmenwand 25 des äußeren U-Rahmenteils 3.

Äußeres und inneres U-Rahmenteil 3, 5 können jeweils in ihrer horizontalen Rahmenwand 22, 24 (d.h. in der Ebene ihrer horizontalen L-Profilschenkel) und insbesondere in deren zur L-Innenseite 20 des L-Profils (vgl. Fig. 5 und Fig. 6) bzw. zur vertikalen Rahmenwand 25, 27 hinweisenden Oberfläche 30, 32 etwa mittig und der U-Form folgend eine oder mehrere längliche Vertiefung(en) (Längsnut(en), Längsrille(n)) 18 aufweisen (vgl. Fig. 1 und Fig. 2). In diesem Fall sind die Löcher 16 für die Nahtführung vorzugsweise in dieser/diesen länglichen Vertiefung(en) 18 angeordnet (siehe Fig. 1 B).

### Mögliche Beschichtung der Hautkontaktflächen des Rahmens:

Die Bereiche des äußeren und inneren U-Rahmenteils 3, 5, die in direktem Kontakt mit den Hauträndern am U-förmigen Hautlappen und/oder mit den Hauträndern entlang der Hautöffnung stehen, insbesondere die Oberflächen der horizontalen Rahmenwände 22, 24 und der vertikalen Rahmenwände 25, 27, die zur L-Innenseite 20 des L-Profils (vgl. Fig. 5 und Fig. 6) hinweisen, können mit einer Beschichtung versehen sein, die aus einem Material besteht, welches die Verhaftung zwischen Rückenhautgewebe und Klapprahmenoberfläche fördert. Diese Beschichtung kann eine aufsprühbare oder aufstreichbare Filmbeschichtung sein, die beispielsweise aus einem bekannten Fibrinkleber oder einem anderen Gewebekleber besteht, und die von Zeit zu Zeit erneuert werden muss. Die Fixierung der Kontaktflächen der U-Rahmenteile am Hautgewebe wird bei dieser Ausführungsvariante durch eine Kombination von chirurgischer Naht und Gewebekleber erreicht.

Alternativ kann die Beschichtung als dauerhaft montierte Beschichtung an der Rahmenoberfläche ausgebildet sein.

Insbesondere die dauerhaft montierte Beschichtung (aber auch die der regelmäßig erneuerungsbedürftigen Beschichtung) kann aus einem Material bestehen, das eine poröse Matrix umfasst, in der die Poren (z.B. über Kanäle) zu einem Netzwerk miteinander verbunden sind (offen poröse Matrix). In diesem Netzwerk haben beispielsweise die Poren einen Durchmesser von 50-200µm und die Kanäle einen Durchmesser von 20- 100µm. Das Material kann ein Polycarbonat, eine Matrix auf Kalzium- oder Kieselsäurebasis oder ein anderes geeignetes bzw. aushärtbares Material sein. Vorzugsweise hat die Außenseite der Beschichtung größere Poren und Anschlüsse als die Innenseite, wodurch das Einwachsen von Gewebebrücken und Blutgefäßen gefördert wird, die an Dicke und Durchmesser abnehmen, wenn sie sich dem "Boden" der Beschichtung nähern.

### Beispiel 2: Installation/Montage/Etablierung eines erfindungsgemäßen Hautkammersystems mit Klapprahmen im/am Versuchstier

Im folgende wird am Beispiel einer Laborratte beschrieben, wie ein erfindungsgemäßes Hautkammersystem mit U-förmigem Klapprahmen in die Rückenhaut eines Versuchstiers chirurgisch eingesetzt werden kann.

### (I) Vorbereitung des Eingriffs

- Anästhesierung der Ratte mit beispielsweise Isofluran.
- Analgesierung der Ratte mit beispielsweise 50 mg/ml Tramadol-Lösung; Aufrechterhaltung ihrer Körpertemperatur z.B. mittels Wärmedecke; Schutz der Tieraugen gegen Austrocknen z.B. mittels Augensalbe.
- Enthaaren z.B. mittels eines Rasierapparates.
- Desinfektion des Rückens, z.B. durch Abwischen mit 70%iger Ethanol-in-WasserLösung und Jodlösung im Wechsel.
- Anzeichnen der Schnittlinie am Rücken. Vorzugsweise wird das innere U-Rahmenteil eines erfindungsgemäßen Klapprahmens als Zeichenvorlage verwendet und dessen Außenumriss mit ca. 1 mm Abstand von der vertikalen Rahmenwand als Markierungslinie für die Schnittführung angezeichnet.
- Abdecken des Tieres mittels chirurgischer Folie. Von diesem Arbeitsschritt an wird mit steriler Technik gearbeitet.

### (II) Einsetzen des Rahmens

- Mit Hilfe eines Skalpells wird die Haut entlang der angezeichneten U-förmigen Markierungslinie eingeschnitten. Bei der Schnittführung wird die Schnittlinie (Schnittspur) entlang der U-Schenkel von der U-Basis aus betrachtet vorzugsweise zunächst exakt entlang der U-Schenkel-Markierungslinie geführt. Kurz vor Erreichen des U-Schenkel-Endabschnitts, in dem beim Klapprahmen die Gelenkverbindung angeordnet ist, wird die Schnittlinie im Winkel zur U-Schenkel-Markierungslinie von dieser und von der U-Öffnung weggeführt. Die Größe des Winkels ist so zu wählen, dass der Abstand zwischen den beiden einander gegenüberliegenden Enden der Schnittlinie weiter ist als der Abstand zwischen den U-Schenkelenden der Markierungslinie und etwa so weit wie der Abstand zwischen den freien Kanten der horizontalen Rahmenwände des äußeren Rahmenteils des Klapprahmens, damit nach dessen Einsetzen die Enden der U-Schenkel des inneren und äußeren Rahmenteils mit den dort angeordneten Gelenkverbindungen von Haut bedeckt sind und nicht von der Schnittnaht.
- Der U-förmige Hautlappen wird in der Ebene der Subkutis (Unterhaut) an oder nahe der Grenze zur darunter liegenden Faszienschicht von dieser Faszienschicht freipräpariert, nach distal aus der umliegenden Haut des Tierrückens herausgeklappt, und vor anhängenden Faszienresten befreit.
- Der Klapprahmen wird in die Hautöffnung eingesetzt. Unter Verwendung von z.B. nichtresorbierbarem 5-0 Monofilament-Faden wird mittels Intrakutan-Nähten das innere U-Rahmenteil mit dem präparativ erzeugten U-förmigen Hautlappen und das äußere U-Rahmenteil mit der Haut an den Rändern der Hautöffnung am Tierrücken vernäht. Zur Fixierung des Hautlappens am inneren U-Rahmenteil werden die Nahtfäden vorzugsweise von proximal (d.h. von "unter der Subkutis") U-förmig durch die Kutis geführt, durch die jeweils nebeneinander liegenden Löcher in der horizontalen Rahmenwand des inneren U-Rahmenteils gezogen und auf der freien, dem äußeren U-Rahmenteil zugewandten Oberfläche der Rahmenwand fest zusammengezogen und verknotet. Die Durchstichstellen werden dabei vorzugsweise so in dem Hautlappen platziert, dass beim Festziehen der Naht gleichzeitig die Haut leicht zur Rahmenwand hingezogen wird. Nach dem Festziehen der Nahten kann die Rahmenwand mit der Auflagefläche der Haut mittels Gewebekleber (z.B. Histoacryl^{™}) verklebt werden. Zu diesem Zweck werden die an der Rahmenwand anliegenden Schnittkanten des U-förmigen Hautlappens mit Hilfe einer feinen Pinzette leicht vom der Rahmenwand abgelöst, der Kleber auf die Kontaktfläche unter der Schnittkante gespritzt und die Haut sofort unter Zuhilfenahme von Pinzette und/oder Finger für ca. 10-30 Sekunden fest an die Rahmenwand gedrückt.
- Vorzugsweise wird an den Schenkelenden des U-förmigen Schnittkanals und distal (oberhalb) des Bereichs, in dem die U-Schenkelenden des Klapprahmens mit der Gelenkverbindung angeordnet sind, zudem eine Schutznaht eingefügt, indem die beiden korrespondierenden Schnittkanten von Hautöffnung und Hautlappen mit einer einfachen Naht und mittels z.B. nichtresorbierbarem 4-0 Monofilament chirurgischem Faden vernäht werden.
- Um ein Zusammenwachsen der Haut über den Klapprahmen hinweg zu verhindern, kann vor dem Schließen des Klapprahmens (d.h. vor dem Einklappen/Einschwenken des inneren U-Rahmenteils mit daran fixiertem Hautlappen in das äußere U-Rahmenteil) auf die vertikale Rahmenwand des inneren U-Rahmenteils ein zuvor (z.B. mit Alkohol) sterilisiertes flexibles Band (z.B. ein Kunststoffband aus Polytetrafluorethylen/PTFE wie das im Handel erhältliche Teflon^{™}) aufgelegt werden. Nach dem Schließen des Klapprahmens sollte der nach außen überstehende Teil dieses Bandes in kurzem Abstand zum Klapprahmen abgeschnitten werden, um zu verhindern, dass das Versuchstier dieses Band später wieder herausreißt und damit den Klapprahmen wieder öffnet.
- Vor dem Schließen des Klapprahmens wird Hautöffnung mit dem eingesetzten offenen Klapprahmen mit Antibiotika-Lösung (z.B. einer 0,024-prozentigen Lösung von Borgal in 0,9% NaCl) gewaschen. Danach wird das innere U-Rahmenteil mit dem anliegenden U-förmigen Hautlappen in das in der Wundöffnung fixierte äußere U-Rahmenteil eingeklappt und somit der Klapprahmen geschlossen. Vorzugsweise werden anschließend die Wundränder mit Sprühpflaster versiegelt.
- Um ein Öffnen der Wunde durch das Versuchstier zu verhindern, sollte diesem ein Beißkragen angelegt werden (z.B. Elisabethan Collar^{™} von Braintree Scientific).
- Die Anästhesierung wird beendet, das Tier zum Aufwachen in einen Käfig mit Wärmequelle (z.B. Infrarotlicht) überführt, und dem Tier wird Trinkwasser mit Schmerzmittel (z.B. Tramadol) und Antibiotikum (z.B. Borgal 0,048 %) zur Verfügung gestellt.

### (IV) Nachsorge und Mikroskopie

Das eingesetzte Hautkammersystem sollte in regelmäßigen Abständen (vorzugsweise mindestens zweimal in der Woche) geöffnet werden, um ein Wiederzusammenwachsen des frei präparierten Hautlappens mit dem Gewebe in der Hautöffnung zu verhindern. Dabei können unter anderem mikroskopische Beobachtungen vorgenommen werden.

Im Folgenden ist eine solche Nachsorge in Kombination mit einer mikroskopischen Visualisierung aktiver Blutgefäße beispielhaft beschrieben:
- Anästhesierung des Versuchstieres, z.B. mittels Isofluran (s.o.) und Platzieren auf einer Wärmematte wie vorstehend beschrieben.
- Anlegen eines Venenkatheters (z.B. an der Schwanzvene) mit heparinisierter Kochsalzlösung für eine Injektion von Wirkstoffen und/oder Plasma-Marker zur Visualisierung von Blutgefäßen. (Dieser Schritt kann unterbleiben, wenn keine mikroskopischen Beobachtungen beabsichtigt sind.)
- Vorsichtiges Öffnen des Hautkammersystems bzw. des Klapprahmens: Das innere U-Rahmenteil wird zunächst nur so weit aus dem äußeren U-Rahmenteil herausgeklappt, bis ein schmaler Spalt zwischen innerem und äußerem U-Rahmenteil sichtbar wird. Durch diesen Spalt wird dann mit einer sterilen chirurgischen Schere und stumpfer Resektion etwaige neu gebildete Gewebebrücken zwischen der Hautlappenunterseite und der Oberseite der Hautöffnung im/am Tierrücken wieder aufgetrennt. Danach wird das innere U-Rahmenteil mit anliegendem Hautlappen langsam vollständig ausgeklappt.
- Mikroskopie: Zur mikroskopischen Beobachtung wird die Ratte auf einer Wärmematte seitlich so platziert, so dass das aufgeklappte innere U-Rahmenteil mit anliegendem Hautlappen auf einem Heiztisch zu liegen kommt. Das Gewebe wird mittels 0,9% Kochsalzlösung konstant feucht gehalten. Zur Homogenisierung der Objektebene kann ein Deckgläschen auf das Gewebe aufgebracht werden. Durch den Schwanzvenen-Katheter wird der Plasma-Marker für die Visualisierung von Blutgefäßen (z.B. 50 µl einer sterilen 10 mg/ml Lösung FITC-Dextran in 09% NaCl) injiziert. Gegebenenfalls können bestimmte Wirkstoffe von Interesse ebenfalls über den Schwanzvenen-Katheter injiziert werden.
- Nachsorge: Falls nicht mikroskopiert wird, wird am anästhesierten Tier lediglich der Klapprahmen geöffnet, zusammengewachsenes Gewebe mittels stumpfer Resektion wieder aufgetrennt, das freipräparierte Gewebe mittels Antibiotikalösung gewaschen, und der Klapprahmen unter Verwendung von flexiblem Band und Sprühpflaster (siehe oben unter III) wieder geschlossen. Danach wird das Tier wieder zum Aufwachen gebracht.

### Beispiel 3: Implantation eines Testobjekts in ein am Versuchstier montiertes (etabliertes/installiertes) Hautkammersystem

Der Klapprahmen des erfindungsgemäßen Hautkammersystems wird ca. 1 Woche vor der geplanten Implantation wie in Bespiel 2 beschrieben in die Rückenhaut des betreffenden Versuchstiers eingesetzt. Nach drei bis sieben Tagen hat sich die Blutversorgung im Gewebe des Hautlappens, der am inneren Klapprahmenteil fixiert ist (vgl. Beispiel 1) normalisiert. Sobald dieser Zustand eingetreten ist, können das oder die Material(ien) oder die Zellen implantiert werden.

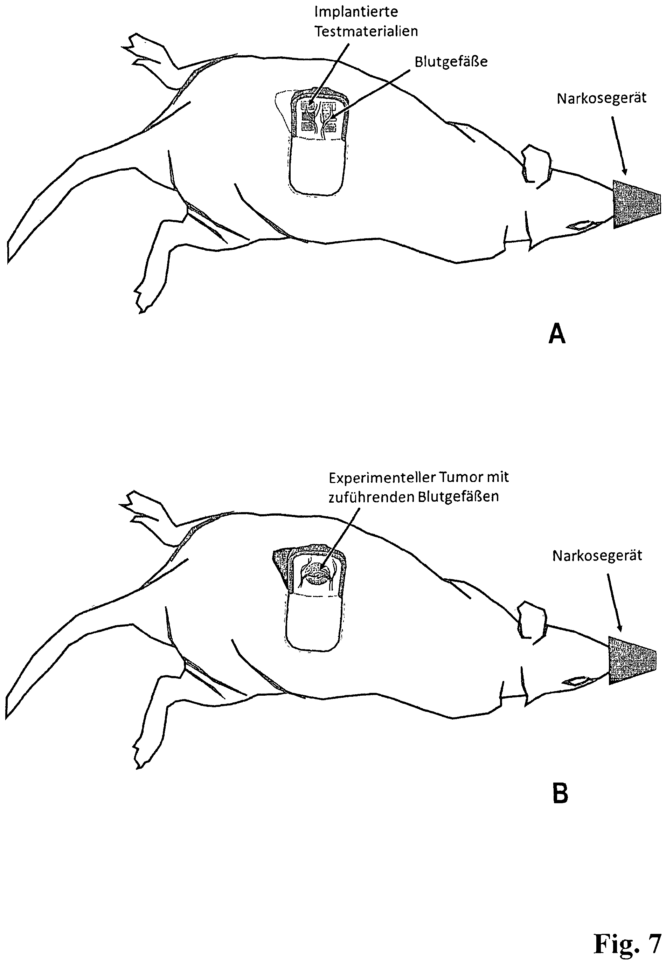

Für die anstehende Implantation des Testobjekts (z.B. Tumorstücke, neuartiges Material) wird das Tier zunächst betäubt. Danach wird der Klapprahmen vorsichtig geöffnet und dabei neu aufgewachsenes Gewebe durch stumpfe Dissektion mit einer Schere entfernt. Anschließend wird die Wunde mit Antibiotika-Lösung gewaschen. Mit feinen chirurgischen Instrumenten wird eine kleine Tasche tangential im exponierten Unterhautgewebe auf der Innenseite des Hautlappens geschaffen (erzeugt). Das zu implantierende Testobjekt wird in die Tasche eingeführt und die Tasche mit einem dünnen Faden (z.B. 6-0 nichtresorbierbarer Monofilamentfaden) vernäht. (Vgl. Fig. 7A, B)

Um zu verhindern, dass die Unterhaut des Hautlappens inklusive Implantat bei geschlossenem Klapprahmen mit der gegenüberliegenden Gewebeseite in der Hautöffnung in/am Tierrücken zusammenwächst und/oder um implantiertes Testobjektgewebe vor Störungen beim Öffnen des Hautlappens zu schützen, wird vorgeschlagen, anstatt des oder zusätzlich zum flexiblen Band(s) (aus vorzugsweise Polytetrafluorethylen / PTFE) ein Stück flexible Folie aus vorzugsweise Polytetrafluorethylen / PTFE oder einem anderen inerten Material als Trennschicht zwischen den Hautlappen und das darunter liegende Gewebe zu legen. Das Stück Folie wird vor dem Schließen derart zwischen die beiden U-Rahmenteile gelegt, dass es beim Schließen des Klapprahmens zwischen dem inneren und dem äußeren U-Rahmenteil und damit zwischen Hautlappen und Tierrücken eingeklemmt wird. Länge und Breite des Folienstücks sind so gewählt, dass die Folien den Klapprahmen bzw. die Rahmenwände und U-Schenkelenden des äußeren Rahmenteils überkragt bzw. überragt, wenn der Klapprahmen geschlossen ist. Der den Klapprahmen überkragende bzw. überragende Rand der Folie kann und sollte knapp über dem Rahmen mittels einer Schere abgeschnitten werden.

Als zu implantierendes Testobjekt kommen insbesondere Materialien oder Zellen in Betracht, die einer Langzeitbeobachtung unter annähernd natürlichen physiologischen und morphologischen Bedingungen unterworfen werden sollen.

Ein wichtiger Faktor für die erfolgreiche Integration von Materialien in ein Gewebe, - z.B. von Knochenersatz oder Herzklappenersatz -, ist die Bildung einer neuen Gefäßversorgung in und um das Implantat herum. Diese Neubildung erfolgt über einen längeren Zeitraum und kann mit Hilfe des erfindungsgemäßen Hautkammersystems mit Klapprahmen gut beobachtet werden.

Das zu implantierende Testobjekt kann insbesondere auch ein Tumorgewebestück sein, das aus einem Tumor stammt, der beispielsweise in einem anderen Wirtstier gewachsen ist und aus diesem extrahiert wurde. Hierzu wird der extrahierte Tumor in sterilem Zellkulturmedium in ca. 2 x 2 mm große Stücke geschnitten, und eines dieser Tumorgewebestücke wird in die im Unterhautgewebe des Hautlappen erzeugte Tasche implantiert. Die Taschenöffnung wird anschließend mit Hilfe eines dünnen Fadens verschlossen.

Nach erfolgter Implantation und dem Schließen des Klapprahmens wird das Tier wie in Beispiel 2 beschrieben zum Aufwachen gebracht.

### Zitierte Nicht-Patent-Literatur:

Palmer GM, Fontanella AN, Shan S, Hanna G, Zhang G, Fraser CL, Dewhirst MW:
   *"*In vivo optical molecular imaging and analysis in mice using dorsal window chamber models applied to hypoxia, vasculature and fluorescent reporters" Nat Protoc. 2011 Aug 18; 6(9): 1355-66. PMID: 21886101
Prunier C, Chen N, Ritsma L, Vrisekoop N: "Procedures and applications of long-term intravital microscopy" Methods. 2017 Sep 1;128:52-64. doi: 10.1016/j.ymeth.2017.06.029. Epub 2017 Jun 30. PMID: 28669866
Sobolik T, Su YJ, Ashby W, Schaffer DK, Well S, Wikswo JP, Zijlstra A, Richmond A: "Development of novel murine mammary imaging windows to examine wound healing effects on leukocyte trafficking in mammary tumors with intravital imaging" IntraVital 2016, Vol. 5, No. 1, e1125562

## Patentansprüche

1. Hautkammersystem mit einer Rahmenvorrichtung, **dadurch gekennzeichnet, dass** die Rahmenvorrichtung als U-förmiger Klapprahmen (1) mit einem U-förmigen äußeren Rahmenteil (3) und einem U-förmigen inneren Rahmenteil (5) realisiert ist,
- wobei diese beiden Rahmenteile (3, 5) an den offenen Enden ihrer U-Schenkel durch je eine Gelenkverbindung (7) derart miteinander gekoppelt sind, dass das innere Rahmenteil (5) gegen das äußere Rahmenteil (3) klappbar ist, so dass im eingeklappten Zustand das innere Rahmenteil (5) innerhalb der U-Öffnung des äußeren Rahmenteils (3) positioniert ist, und im ausgeklappten Zustand das innere Rahmenteil (5) und das äußeren Rahmenteil (3) spiegelbildlich zueinander liegen und eine Öffnung umschließen,
- und wobei äußeres Rahmenteil (3) und inneres Rahmenteil (5) im Querschnitt jeweils ein L-förmiges Profil haben, so dass jedes Rahmenteil (3, 5) eine bezüglich der Klapprahmenebene (8) horizontale U-förmig verlaufende Rahmenwand (22, 24) und eine bezüglich der Klapprahmenebene (8) vertikale U-förmig verlaufende Rahmenwand (25, 27) aufweist,
- und wobei diese beiden Rahmenteile (3, 5) derart zueinander ausgerichtet sind, dass im geschlossenen Klapprahmen (1) ihre vertikalen Rahmenwände (25, 27) annähernd parallel zu einander verlaufen und ihre horizontalen Rahmenwände (22, 24) im wesentlichen in der gleichen Ebene und gegenläufig zueinander ausgerichtet sind.

2. Hautkammersystem nach Anspruch 1, **dadurch gekennzeichnet, dass** die Gelenkverbindung (7) derart an den Enden der U-Schenkel des inneren und äußeren Rahmenteils (5, 3) angeordnet ist, dass beim Öffnen des Klapprahmens (1) durch Ausklappen des inneren Rahmenteils (5) aus dem äußeren Rahmenteil (3) oder beim Schließen des Klapprahmens (1) durch Einklappen des inneren Rahmenteils (5) in das äußere Rahmenteil (3) die Enden der U-Schenkel des inneren Rahmenteils (5) die Enden der U-Schenkel und die Rahmenwände (22, 25) des äußeren Rahmenteils (3) nicht überkragen.

3. Hautkammersystem nach Anspruch 1 oder 2, **dadurch gekennzeichnet, dass** die Gelenkverbindung (7) ein Scharnier ist, welches an den beiden vertikalen Rahmenwänden (25, 27) von äußerem und innerem Rahmenteil (3, 5) ausgebildet ist, und dass das Scharnier (7) zwei Ösen und einen Verbindungsstift (21) aufweist, wobei die Ösen jeweils als eine Lochbohrung (17, 19) in der vertikalen Rahmenwand (27) des inneren Rahmenteils (5) und in der vertikalen Rahmenwand (25) des äußeren Rahmenteils (3) und jeweils nahe der freien U-Schenkelenden realisiert sind, und wobei der Verbindungsstift (21) in den beiden Ösen derart angeordnet und ausgebildet ist, dass er nicht über den von den beiden Ösen gebildeten Stiftkanal hinausragt.

4. Hautkammersystem nach einem der Ansprüche 1 bis 3 **dadurch gekennzeichnet, dass** das äußere und/oder das innere Rahmenteil (3, 5) in der horizontalen Rahmenwand (22, 24) Löcher/Durchgangsbohrungen (16) aufweist, die einzeln oder gruppiert angeordnet sind.

5. Hautkammersystem nach einem der Ansprüche 1 bis 4, **dadurch gekennzeichnet, dass** die horizontale Rahmenwand (22) des äußeren Rahmenteils (3) und/oder die horizontale Rahmenwand (24) des inneren Rahmenteils (5) in ihrer zur L-Innenseite (20) des L-Profils im Querschnitt hinweisenden Oberfläche (30, 32) etwa mittig und der U-Form folgend wenigstens eine längliche Vertiefung (Längsnut, Längsrille) (18) aufweist.

6. Hautkammersystem nach einem der Ansprüche 1 bis 5, **dadurch gekennzeichnet, dass** das Material des äußeren und inneren Rahmenteils (3, 5) und der Gelenkverbindung ausgewählt ist aus der Gruppe bestehend aus: Edelstahl, Titan und biokompatiblen Kunststoffen wie insbesondere Polyethylen (PE), Polyvinylchlorid (PVC), Acrylglas (PMMA), Polycarbonat (PC), sowie Kombinationen davon.

7. Hautkammersystem nach einem der Ansprüche 1 bis 6, **dadurch gekennzeichnet, dass** an denjenigen Oberflächen der horizontalen Rahmenwände (22, 24) und/oder der vertikalen Rahmenwände (25, 27), die zur L-Innenseite (20) des L-Profils des äußeren und/oder inneren Rahmenteils (3, 5) im Querschnitt hinweisen, eine Beschichtung ausgebildet ist, die aus einem Material besteht, welches die Verhaftung zwischen Hautgewebe und Rahmenwand fördert.

8. Hautkammersystem nach Anspruch 7, **dadurch gekennzeichnet, dass** das Material der Beschichtung eine poröse Matrix auf der Basis von Polycarbonat und/oder Kalzium und/oder Kieselsäure umfasst, deren Poren über Kanäle miteinander verbunden sind.

9. Hautkammersystem nach einem der Ansprüche 1 bis 8, **dadurch gekennzeichnet, dass** das äußere Rahmenteil (3) an seiner horizontalen Rahmenwand (22) im Bereich der U-Basis (2) des Klapprahmens (1) eine laschenartige Auskragung (4) aufweist.

10. Hautkammersystem nach einem der Ansprüche 1 bis 9, **dadurch gekennzeichnet, dass** das innere Rahmenteil (5) an seiner vertikalen Rahmenwand (27) im Bereich der U-Basis (2) des Klapprahmens (1) eine laschenartige Auskragung (6) aufweist.

11. Hautkammersystem nach einem der Ansprüche 1 bis 10 zur Anwendung in einem mikroskopischen oder nicht-mikroskopischen Verfahren zur Beobachtung, Überwachung, Detektion und/oder Messung von physiologischen Zuständen in vivo.

## Claims

1. Skin chamber system with a frame device, **characterized in that**
- the frame device is implemented as a U-shaped folding frame (1) with a U-shaped outer frame part (3) and a U-shaped inner frame part (5)
- wherein these two frame parts (3, 5) are coupled to one another at the open ends of their U legs by a respective articulated connection (7) in such a way that the inner frame part (5) can be folded against the outer frame part (3), so that in the folded-in state the inner frame part (5) is positioned inside the U opening of the outer frame part (3), and in the unfolded state the inner frame part (5) and the outer frame part (3) lie mirror-inverted to one another and enclose an opening,
- and wherein the outer frame part (3) and the inner frame part (5) each have an L-shaped profile in cross-section, so that each frame part (3, 5) has a U-shaped frame wall (22, 24) extending horizontally with respect to the folding frame plane (8) and a U-shaped frame wall (25, 27) extending vertically with respect to the folding frame plane (8),
- and these two frame parts (3, 5) being aligned with respect to one another in such a way that, in the closed folding frame (1), their vertical frame walls (25, 27) run approximately parallel to one another and their horizontal frame walls (22, 24) are aligned essentially in the same plane and in opposite directions to one another.

2. Skin chamber system according to claim 1, **characterized in that** the articulated connection (7) is arranged at the ends of the U legs of the inner and outer frame parts (5, 3) in such a way, that when the folding frame (1) is opened by folding the inner frame part (5) out of the outer frame part (3) or when the folding frame (1) is closed by folding the inner frame part (5) into the outer frame part (3), the ends of the U legs of the inner frame part (5) do not overhang the ends of the U legs and the frame walls (22, 25) of the outer frame part (3).

3. Skin chamber system according to claim 1 or 2, **characterized in that** the hinge (7) is a hinge formed on the two vertical frame walls (25, 27) of outer and inner frame parts (3, 5), and **in that** the hinge (7) has two eyelets and a connecting pin (21), the eyelets each being formed as a drilled hole (17, 19) in the vertical frame wall (27) of the inner frame part (5) and in the vertical frame wall (25) of the outer frame part (3), respectively, in the proximity of the free U-leg ends, and the connecting pin (21) is arranged and formed in the two eyelets in such a way that it does not project beyond the pin channel formed by the two eyelets.

4. Skin chamber system according to any one of claims 1 to 3 **characterized in that** the outer and/or the inner frame part (3, 5) has holes/through-holes (16) in the horizontal frame wall (22, 24), which are arranged individually or grouped.

5. Skin chamber system according to one of the claims 1 to 4, **characterized in that** the horizontal frame wall (22) of the outer frame part (3) and/or the horizontal frame wall (24) of the inner frame part (5) has, in its surface (30, 32) facing the L inner side (20) of the L profile in cross-section, approximately centrally and following the U shape, at least one elongated depression (longitudinal slot, longitudinal groove) (18).

6. Skin chamber system according to any of claims 1 to 5, **characterized in that** the material of the outer and inner frame part (3, 5) and of the hinge connection is selected from the group consisting of: Stainless steel, titanium and biocompatible plastics such as in particular polyethylene (PE), polyvinyl chloride (PVC), acrylic glass (PMMA), polycarbonate (PC), and combinations thereof.

7. A skin chamber system according to any one of claims 1 to 6, **characterized in that** on those surfaces of the horizontal frame walls (22, 24) and/or the vertical frame walls (25, 27) facing the L-inner side (20) of the L-profile of the outer and/or inner frame part (3, 5) in cross-section, a coating is formed which consists of a material which promotes adhesion between skin tissue and frame wall.

8. Skin chamber system according to claim 7, **characterized in that** the material of the coating comprises a porous matrix based on polycarbonate and/or calcium and/or silica, the pores of which are interconnected by channels.

9. Skin chamber system according to one of claims 1 to 8, **characterized in that** the outer frame part (3) has a tab-like overhang (4) on its horizontal frame wall (22) in the region of the U base (2) of the folding frame (1).

10. Skin chamber system according to one of claims 1 to 9, **characterized in that** the inner frame part (5) has a tab-like overhang (6) on its vertical frame wall (27) in the region of the U base (2) of the folding frame (1).

11. Skin chamber system according to any one of claims 1 to 10 for use in a microscopic or non-microscopic method for observing, monitoring, detecting and/or measuring physiological conditions in vivo.

## Revendications

1. Système de chambre cutanée avec un dispositif de cadre, **caractérisé en ce que** le dispositif de cadre est réalisé sous la forme d'un cadre pliable (1) en forme de U avec une partie de cadre extérieure (3) en forme de U et une partie de cadre intérieure (5) en forme de U,
- ces deux parties de cadre (3, 5) étant couplées l'une à l'autre à chaque extrémité ouverte de leurs branches en U par une liaison articulée (7) de telle sorte que la partie de cadre intérieure (5) peut être rabattue contre la partie de cadre extérieure (3) de sorte que, à l'état rabattue, la partie de cadre intérieure (5) est positionnée à l'intérieur de l'ouverture en U de la partie de cadre extérieure (3) et, à l'état déplié, la partie de cadre intérieure (5) et la partie de cadre extérieure (3) sont disposées en image miroir l'une par rapport à l'autre et entourent une ouverture,
- et la partie de cadre extérieure (3) et la partie de cadre intérieure (5) ayant chacune en coupe transversale un profil en forme de L, de sorte que chaque partie de cadre (3, 5) présente une paroi de cadre (22, 24) en forme de U horizontale par rapport au plan du cadre pliable (8) et une paroi de cadre (25, 27) en forme de U verticale par rapport au plan du cadre pliant (8),
- et ces deux parties de cadre (3, 5) étant orientées l'une par rapport à l'autre de telle sorte que, dans le cadre pliable (1) fermé, leurs parois de cadre verticales (25, 27) s'étendent de façon sensiblement parallèle l'une par rapport à l'autre, et leurs parois de cadre horizontales (22, 24) sont orientées sensiblement dans le même plan et en sens opposé l'une de l'autre.

2. Système de chambre cutanée selon la revendication 1, **caractérisé en ce que** la liaison articulée (7) est disposée de telle sorte aux extrémités des branches en U des parties de cadre intérieure et extérieure (5, 3), que lors de l'ouverture du cadre pliable (1) en dépliant la partie de cadre intérieure (5) hors de la partie de cadre extérieure (3), ou lors de la fermeture du cadre pliable (1) en rabattant la partie de cadre intérieure (5) dans la partie de cadre extérieure (3), les extrémités des branches en U de la partie de cadre intérieure (5) ne dépassent pas les extrémités des branches en U et les parois de cadre (22, 25) de la partie de cadre extérieure (3).

3. Système de chambre cutanée selon la revendication 1 ou 2, **caractérisé en ce que** la liaison articulée (7) est une charnière qui est formée sur les deux parois de cadre verticales (25, 27) des parties de cadre extérieure et intérieure (3, 5), et **en ce que** la charnière (7) comprend deux œillets et une broche de liaison (21), les œillets étant chacun un trou perforé (17, 19) dans la paroi de cadre verticale (27) de la partie de cadre intérieure (5) et dans la paroi de cadre verticale (25) de la partie de cadre extérieure (3) et sont réalisés respectivement à proximité des extrémités libres des branches en U, et la broche de liaison (21) étant disposée dans les deux œillets et réalisée de telle sorte qu'elle ne dépasse pas du canal de broche formé par les deux œillets.

4. Système de chambre cutanée selon l'une des revendications 1 à 3, **caractérisé en ce que** la partie de cadre extérieure et/ou intérieure (3, 5) présente des trous/perçages de passage (16) dans la paroi de cadre horizontale (22, 24), qui sont disposés de façon individuelle ou en groupe.

5. Système de chambre cutanée selon l'une des revendications 1 à 4, **caractérisé en ce que** la paroi de cadre horizontale (22) de la partie de cadre extérieure (3) et/ou la paroi de cadre horizontale (24) de la partie de cadre intérieure (5) présente dans sa surface (30, 32) orientée en coupe transversale vers le côté intérieur en L (20) du profilé en L, au moins un renfoncement allongé (rainure longitudinale, sillon longitudinal) (18) à peu près au milieu et suivant la forme en U.

6. Système de chambre cutanée selon l'une des revendications 1 à 5, **caractérisé en ce que** le matériau de la partie de cadre extérieure et intérieure (3, 5) et de la liaison articulée est choisi dans le groupe constitué de : l'acier inoxydable, le titane et des matières plastiques biocompatibles, comme notamment le polyéthylène (PE), le polychlorure de vinyle (PVC), le verre acrylique (PMMA), le polycarbonate (PC), ainsi que leurs combinaisons.

7. Système de chambre cutanée selon l'une des revendications 1 à 6, **caractérisé en ce qu'**un revêtement constitué d'un matériau favorisant l'adhérence entre le tissu cutané et la paroi de cadre est formé sur les surfaces des parois de cadre horizontales (22, 24) et/ou des parois de cadre verticales (25, 27) qui sont orientées en coupe transversale vers le côté intérieur en L (20) du profilé en L de la partie de cadre extérieure et/ou intérieure (3, 5), est formé.

8. Système de chambre cutanée selon la revendication 7, **caractérisé en ce que** le matériau du revêtement comprend une matrice poreuse à base de polycarbonate et/ou de calcium et/ou de l'acide silicique, dont les pores sont reliés entre eux par des canaux.

9. Système de chambre cutanée selon l'une des revendications 1 à 8, **caractérisé en ce que** la partie de cadre extérieure (3) présente une saillie (4) de type languette sur sa paroi de cadre horizontale (22) au niveau de la base du U (2) du cadre pliable (1).

10. Système de chambre cutanée selon l'une des revendications 1 à 9, **caractérisé en ce que** la partie de cadre intérieure (5) présente une saillie (6) de type languette sur sa paroi de cadre verticale (27) au niveau de la base du U (2) du cadre pliable (1).

11. Système de chambre cutanée selon l'une des revendications 1 à 10, destiné à être utilisé dans un procédé microscopique ou non microscopique d'observation, de surveillance, de détection et/ou de mesure d'états physiologiques in vivo.
